# DEMANDE DE BREVET EUROPEEN

(11) **EP 3 797 625 A1**
(43) Date de publication de la demande: **31.03.2021**
(21) Numéro de dépôt: 20169506.1
(22) Date de dépôt: 14.04.2020
(51) Int. Cl.: A43B 3/00, A43B 13/00, A43B 17/00, A43B 13/18, A43D 999/00

(54) **PROCEDE ET SYSTEME POUR LE CALCUL DE VALEURS PERSONNALISEES DE PARAMETRES D'UNE SEMELLE POUR LA CONCEPTION DE SEMELLES SUR MESURE**

(30) Priorité: 24.09.2019 EP 19306195
(71) Demandeur: Zhor Tech, 54000 Nancy (FR)
(72) Inventeur: OUMNIA, Karim, 54000 Nancy (FR)
(74) Mandataire: A.P.I. Conseil

(57) **Abrégé**

L'invention porte sur un procédé (500) de calcul de valeurs personnalisées de paramètres d'une nouvelle semelle pour la conception de semelles sur mesure, ledit procédé comprenant :
- une étape de chargement (530) de valeurs de paramètres de posture ou de mobilité (101) d'un utilisateur ;
- une étape de chargement (540) de valeurs de paramètres de chaussure (201);
- une étape de calcul (550) d'une ou de plusieurs valeurs personnalisées de paramètres d'une nouvelle semelle (301) ;
- une étape de réception et d'analyse (560) d'informations d'utilisateurs tiers d'articles chaussants tiers (11'), lesdites informations d'utilisateurs tiers comportant pour chaque utilisateur tiers d'articles chaussants tiers :
∘ des valeurs de paramètres de posture ou de mobilité tiers (101 '), et
∘ des valeurs de paramètres de chaussure tiers (201') de l'article chaussant tiers (11') ;

- une étape d'identification (580) de valeurs de paramètres de semelle ajustées.

## Description

L'invention concerne le domaine des articles chaussants et de leur conception, l'invention peut trouver une application dans le suivi d'activités quotidiennes ou sportives, ou encore le suivi de l'état physiologique du sujet d'étude de façon à déterminer des valeurs personnalisées de paramètres d'une semelle pour la conception de semelles sur-mesure. L'invention porte sur un procédé de calcul de valeurs personnalisées de paramètres d'une semelle pour la conception de semelles sur mesure.

### [Art antérieur]

Le pied constitue une partie du corps humain particulièrement complexe de par sa composition puisqu'il comporte 26 os, 107 ligaments et près de 19 muscles. Il revêt également un rôle particulièrement important puisqu'il est la clef de voûte permettant à un être humain de se mouvoir. Le moindre inconfort ou la moindre dégradation de ce dernier peut rapidement être handicapante. Cela est particulièrement vrai dans le cadre de la pratique d'un sport impliquant un contact du pied avec le sol, que l'on retrouve dans une grande partie des sports terrestres. Dans le cadre de ces sports, des blessures au pied peuvent survenir à suite à d'une mauvaise pratique. Cependant, des blessures peuvent également survenir à suite à d'une utilisation inadaptée d'articles chaussants, c'est-à-dire ne présentant pas les caractéristiques techniques (amorti, flexibilité, semelle destinée à un type de foulée particulier etc.) adaptées à la pratique du sport par la personne. De nos jours, le consommateur est confronté à une variété de choix et de sélections d'articles chaussants qui n'étaient pas disponibles auparavant. Bien que des morphologies standards de pieds humains existent, il n'en demeure pas moins que chaque pied est unique et peut ne pas correspondre aux standards existants. Ainsi l'utilisation d'articles chaussants peu ou pas adaptés au pied d'une personne, aura tendance à entrainer à moyen ou long terme des dégradations des pieds de la personne. Historiquement, il a été développé des orthèses plantaires pour corriger une posture, un déséquilibre biomécanique, des jambes de longueur inégale pouvant entrainer une scoliose, ou encore des douleurs d'arthrose du genou. Outre les orthèses plantaires, des articles chaussants sur mesure peuvent également être confectionnés, généralement par un spécialiste de santé. Pour cela, une procédure bien connue pour réaliser des articles chaussants consiste à effectuer une mesure sur le pied du futur porteur de l'article chaussant afin de déterminer tous les paramètres morphométriques du pied. En cas de mauvais positionnement du pied, une coulée ou empreinte du pied est alors réalisée, selon laquelle un insert de chaussure orthopédique est réalisé. Au moyen des dimensions déterminées du pied, un flan de cuir plat est coupé à la taille. Un modèle de pied dimensionné est ensuite réalisé. Le flan de cuir est ensuite étiré sur ce modèle et la semelle est collée dessus, généralement par couture et / ou collage. Ce processus de fabrication, artisanal, est particulièrement compliqué et long à mettre en œuvre, en particulier lorsqu'un modèle de réplication du pied est produit. De même, la production d'un article chaussant ajustée prend entre 4 et 6 semaines et nécessite l'intervention de spécialistes de santé. Ainsi, afin de fournir un article chaussant adapté au pied d'un utilisateur, une solution, décrite dans la demande de brevet US10/148,700, a été développée. La solution ainsi décrite porte sur un procédé de fabrication d'articles chaussants ajustés, ledit procédé comprenant l'utilisation d'une semelle préparée, d'une tige préformée et d'un insert. La semelle préfabriquée présente des dimensions correspondant à celles du pied destiné à porter l'article chaussant. L'insert est alors appliqué sur la semelle préfabriquée et l'ajustement entre la semelle préfabriquée et l'insert qui lui est appliqué est vérifié sur le pied destiné à porter l'article chaussant. La tige préformée est placée sur le pied destiné à porter l'article chaussant et est jointe à la semelle préfabriquée de manière à former un raccord. Cependant cette solution ne permet pas de prendre en compte, outre les paramètres morphométriques des pieds du porteur, des paramètres associés à la démarche dudit porteur.

Une autre solution décrite dans la demande de brevet CN105243547 vise à fournir une plateforme de service de personnalisation d'articles chaussants. Une telle plateforme comprend ainsi un système de collecte d'informations, un système de traitement de données et un système de fabrication. Le système de collecte d'informations est configuré pour collecter des données de démarche de l'utilisateur, des données tridimensionnelles du pied et des informations de sélection de chaussure, et envoyer les données de démarche de l'utilisateur, des données tridimensionnelles du pied et des informations de sélection de chaussure à un système de traitement de données. Le système de collecte d'informations consiste principalement en une pluralité de capteurs placés au sein de la semelle intérieure de l'article chaussant. Le système de traitement de données est utilisé pour analyser et traiter les données de démarche de l'utilisateur, les données tridimensionnelles de forme de pied et les informations de sélection de chaussures, et envoyer le résultat d'analyse et de traitement à un système de fabrication. Concernant les informations de sélection de chaussure, l'utilisateur sélectionne une marque en ligne en fonction de ses propres besoins, il sélectionne également le style et le matériau de la chaussure. Cependant, cette solution demeure incomplète puisqu'elle ne prend en compte que la pression exercée par le porteur de l'article chaussant, et ne propose pas une solution complétement intégrée à la semelle de l'article chaussant.

Les méthodes de l'art antérieur ne sont basées que sur des analyses incomplètes de la démarche d'un utilisateur d'articles chaussants. En outre, les informations récoltées sont généralement parcellaires et ne permettent pas de contextualiser lesdites informations.

Ainsi, les solutions existantes ne prennent pas en compte l'évolution au cours du temps de la posture et de la mobilité d'un individu notamment en lien avec les semelles qu'il utilise. Il existe donc un besoin pour de nouvelles solutions permettant de proposer des articles chaussants personnalisés à un utilisateur, en prenant en compte d'une part la démarche de l'utilisateur et d'autre part les paramètres de l'article chaussant porté par ce dernier.

### [Problème technique]

L'invention a donc pour but de remédier aux inconvénients de l'art antérieur. En particulier, l'invention a pour but de proposer un procédé de calcul de valeurs personnalisées de paramètres d'une semelle pour la conception de semelles sur mesure, en prenant en considération les paramètres de posture ou de mobilité de l'utilisateur et les paramètres de l'article chaussant porté par ce dernier.

### [Brève description de l'invention]

L'invention porte notamment sur un procédé de calcul de valeurs personnalisées de paramètres d'une nouvelle semelle pour la conception de semelles sur mesure, ledit procédé de calcul comportant l'exécution, par un ou plusieurs dispositifs informatiques, des étapes suivantes :
- une étape de chargement de valeurs de paramètres de posture ou de mobilité d'un utilisateur, lesdites valeurs de paramètres de posture ou de mobilité ayant été calculées à partir de données brutes générées par au moins une semelle connectée associée à un article chaussant utilisé par ledit utilisateur ;
- une étape de chargement de valeurs de paramètres de chaussure, lesdites valeurs de paramètres de chaussure comportant des valeurs de paramètres structurels et/ou géométriques de l'article chaussant utilisé par l'utilisateur, associé à la semelle connectée ;
- une étape de calcul d'une ou de plusieurs valeurs personnalisées de paramètres d'une nouvelle semelle, la ou lesdites valeurs personnalisées de paramètres de la nouvelle semelle étant calculées à partir des valeurs de paramètres de posture ou de mobilité et des valeurs de paramètres de chaussure ;
- une étape de réception et d'analyse d'informations d'utilisateurs tiers d'articles chaussants tiers, lesdites informations d'utilisateurs tiers comportant pour chaque utilisateur tiers d'articles chaussants tiers :
   ∘ des valeurs de paramètres de posture ou de mobilité tiers calculées à partir de données brutes générées par au moins une semelle connectée tierce associée à un article chaussant tiers, et
   ∘ des valeurs de paramètres de chaussure tiers de l'article chaussant tiers associé à l'au moins une semelle connectée tierce ;
- une étape d'identification de valeurs de paramètres de semelle ajustées, ladite étape d'identification comportant une comparaison des valeurs de paramètres de posture ou de mobilité générées par la semelle connectée et des paramètres de chaussure de l'article chaussant associé à ladite semelle connectée, aux informations d'utilisateurs tiers préalablement analysées.

Ainsi, un procédé selon l'invention sera à même de proposer des valeurs de paramètres de semelles réellement adaptées à un utilisateur en fonction de données directement générées lors de ses déplacements mais également en fonction de données issues d'autres utilisateurs. Cela permet d'améliorer encore le niveau de personnalisation et de performance tout en bénéficiant d'une information sourcée auprès d'autres utilisateurs pouvant présenter des démarches similaires.

**Selon d'autres caractéristiques optionnelles du procédé, ce dernier peut inclure facultativement une ou plusieurs des caractéristiques suivantes, seules ou en combinaison :**
- les informations d'utilisateurs tiers comportent en outre une valeur d'indice de satisfaction d'un utilisateur tiers, de préférence vis-à-vis de la semelle connectée tierce et/ou de l'article chaussant comportant ladite semelle connectée tierce. La présence d'un tel indice puis éventuellement son utilisation lors d'une étape d'identification de valeurs de paramètres de semelle permet par exemple de ne sélectionner que les valeurs associées à des valeurs d'indice supérieures à un seuil prédéterminé ou encore d'affecter un coefficient de pondération en fonction de la valeur de l'indice de satisfaction. Ainsi, les valeurs de paramètres de la nouvelle semelle en sont améliorées de même que la satisfaction de l'utilisateur. Par exemple, l'étape d'identification de valeurs de paramètres de semelle ajustées peut être mise en oeuvre uniquement pour les informations d'utilisateurs tiers comportant une valeur d'indice de satisfaction et/ou un indice de corrélation respectivement supérieur(s) à un seuil prédéterminé.
- le procédé comprend une étape de détermination d'un indice de corrélation, pour chaque semelle connectée tierce, entre les valeurs de paramètre de posture ou de mobilité tiers, les valeurs de paramètres de chaussure tiers et l'indice de satisfaction de l'utilisateur tiers. Une telle étape permet d'améliorer les valeurs de paramètres de la nouvelle semelle de même que la satisfaction de l'utilisateur. En effet, peuvent être pris en compte que les avis d'utilisateurs tiers dont l'utilisation est en adéquation avec l'utilisation préconisée pour une semelle connectée tierce équipant un article chaussant. Il est ainsi possible de mettre en évidence les semelles connectées ou les articles chaussants présentant des paramètres de chaussure considérés comme les plus adaptés et pour une utilisation conforme.
- Le procédé comporte l'étape d'identification de valeurs de paramètres de semelle ajustées est mise en oeuvre uniquement pour les informations d'utilisateurs tiers comportant une valeur d'indice de satisfaction et/ou un indice de corrélation respectivement supérieur(s) à un seuil prédéterminé. Cela permet avantageusement de ne pas prendre en compte les valeurs de paramètres provenant d'articles chaussants ou de semelles tiers potentiellement inadaptées à l'utilisateur.
- le procédé comprend une étape de traitement, préalable à l'étape de chargement, de données brutes générées par l'au moins une semelle connectée utilisée par l'utilisateur, ladite étape de traitement permettant la génération de valeurs de paramètres de posture ou de mobilité. Cette étape de traitement peut être réalisée au niveau des boitiers, au niveau d'un dispositif mobile et/ou au niveau d'un serveur.
- les paramètres de posture ou de mobilité sont sélectionnés parmi la force d'impact au contact au sol, les paramètres de pronation et/ ou supination et/ou une boiterie. Ces paramètres lorsqu'ils sont utilisés permettent la génération de valeurs de paramètres de la nouvelle semelle particulièrement adaptés.
- Le procédé comporte une étape de génération d'une valeur d'un premier indicateur de confort d'une première semelle, ledit premier indicateur de confort étant généré suite à la saisie de données par l'utilisateur, par l'intermédiaire d'une interface homme-machine d'un dispositif informatique de présentation et en ce que l'étape de calcul d'une ou de plusieurs valeurs personnalisées de paramètres de la nouvelle semelle, prend en compte la valeur générée du premier indicateur de confort. Une telle étape permet d'améliorer les valeurs de paramètres de la nouvelle semelle de même que la satisfaction de l'utilisateur.
- Le procédé comporte une étape de génération d'une valeur d'un deuxième indicateur de confort d'une deuxième semelle, ledit deuxième indicateur de confort étant généré suite à la saisie de données par l'utilisateur de ladite deuxième semelle, par l'intermédiaire de l'interface homme-machine du dispositif informatique de présentation et en ce que l'étape de calcul d'une ou de plusieurs valeurs personnalisées de paramètres de la nouvelle semelle, prend en compte la valeur générée du deuxième indicateur de confort. Une telle étape permet d'améliorer les valeurs de paramètres de la nouvelle semelle de même que la satisfaction de l'utilisateur.
- l'étape de calcul d'une ou de plusieurs valeurs personnalisées de paramètres de la nouvelle semelle, comprend en outre une comparaison des valeurs de paramètres de posture ou de mobilité et des valeurs de paramètres de chaussure préalablement chargées aux informations d'utilisateurs tiers. Une telle étape permet d'améliorer les valeurs de paramètres de la nouvelle semelle de même que la satisfaction de l'utilisateur.
- l'étape d'identification de valeurs de paramètres de semelle ajustées comporte l'utilisation d'un référentiel précisant des valeurs cibles ou des plages cibles pour les valeurs personnalisées de paramètres de la nouvelle semelle en fonction des valeurs de paramètres de posture ou de mobilité générées et des valeurs de paramètres de chaussure acquis. Une telle étape permet d'améliorer la satisfaction de l'utilisateur.
- il comporte une étape de calcul d'au moins une valeur de paramètre d'usage à partir des valeurs de paramètres de posture ou de mobilité et en ce que l'étape d'identification de valeurs de paramètres de semelle ajustées prend en compte l'au moins une valeur de paramètre d'usage calculée. Une telle étape permet d'améliorer les valeurs de paramètres de la nouvelle semelle de même que la satisfaction de l'utilisateur.
L'invention porte également sur un procédé de fabrication d'une nouvelle semelle sur mesure par un dispositif d'impression en trois dimensions, ledit procédé comportant les étapes suivantes :
- Téléchargement, par le dispositif d'impression, d'un fichier de configuration comportant une ou plusieurs valeurs personnalisées de paramètres de la nouvelle semelle calculées lors de la mise en oeuvre d'un procédé de calcul de valeurs personnalisées de paramètres d'une nouvelle semelle selon l'invention ;
- Génération, à partir des valeurs personnalisées de paramètres de la nouvelle semelle, d'une maquette numérique de la nouvelle semelle ;
- Impression d'une nouvelle semelle à partir de la maquette numérique générée.

**Selon un troisième aspect**, l'invention porte sur un système de calcul de valeurs personnalisées de paramètres d'une nouvelle semelle pour la conception de semelles sur mesure, ledit système comprenant au moins un dispositif informatique de calcul et un article chaussant comprenant une semelle connectée, ledit au moins un dispositif informatique de calcul étant configuré pour charger des valeurs de paramètres de posture ou de mobilité calculées à partir de données brutes générées par au moins une semelle connectée d'un article chaussant utilisé par un utilisateur, charger des valeurs de paramètres de chaussures, calculer une ou plusieurs valeurs personnalisées de paramètres d'une nouvelle semelle à partir des valeurs de paramètres de posture ou de mobilité et des valeurs de paramètres de chaussure, recevoir et analyser des informations d'utilisateurs tiers d'articles chaussants tiers équipés de semelles connectées tierces, et, identifier des valeurs de paramètres de semelle ajustées.

**Selon un quatrième aspect**, l'invention porte sur un programme informatique comprenant des instructions de programme qui, lorsqu'elles sont exécutées par une unité de traitement d'un dispositif informatique, provoquent la mise en oeuvre d'un procédé de calcul de valeurs personnalisées de paramètres d'une nouvelle semelle pour la conception de semelles sur mesure selon l'invention.

D'autres avantages et caractéristiques de l'invention apparaitront à la lecture de la description suivante donnée à titre d'exemple illustratif et non limitatif, en référence aux Figures annexées :
La Figure 1 représente un schéma illustratif d'un système au sein duquel un procédé de calcul de valeurs personnalisées de paramètres d'une semelle pour la conception de semelles sur mesure conforme à l'invention peut être mis en œuvre.
La Figure 2 représente un système d'analyse d'usage d'articles chaussants utilisé dans le cadre de l'invention et détaillant un boitier électronique d'une semelle connectée.
La Figure 3 représente un schéma illustratif d'un procédé de calcul de valeurs personnalisées de paramètres d'une semelle pour la conception de semelles sur mesure conforme à l'invention.

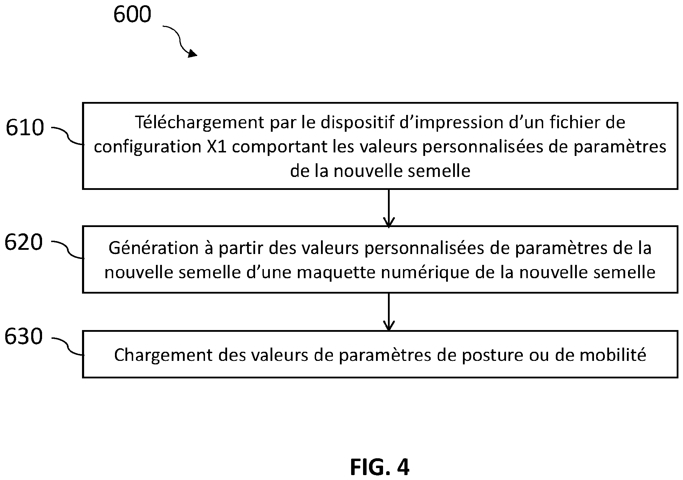

La Figure 4 représente un schéma illustratif d'un procédé de fabrication d'une semelle sur mesure conforme à l'invention par un dispositif d'impression en trois dimensions.

Des aspects de la présente invention sont décrits en référence à des organigrammes et / ou à des schémas fonctionnels de procédés, d'appareils (systèmes) et de produits de programme d'ordinateur selon des modes de réalisation de l'invention.

Sur les figures, les organigrammes et les schémas fonctionnels illustrent l'architecture, la fonctionnalité et le fonctionnement d'implémentations possibles de systèmes, de procédés et de produits de programme d'ordinateur selon divers modes de réalisation de la présente invention. A cet égard, chaque bloc dans les organigrammes ou blocs-diagramme peut représenter un système, un dispositif, un module ou un code, qui comprend une ou plusieurs instructions exécutables pour mettre en œuvre la ou les fonctions logiques spécifiées. Dans certaines implémentations, les fonctions associées aux blocs peuvent apparaître dans un ordre différent que celui indiqué sur les figures. Par exemple, deux blocs montrés successivement peuvent, en fait, être exécutés sensiblement simultanément, ou les blocs peuvent parfois être exécutés dans l'ordre inverse, en fonction de la fonctionnalité impliquée. Chaque bloc des schémas de principe et / ou de l'organigramme, et des combinaisons de blocs dans les schémas de principe et / ou l'organigramme, peuvent être mis en œuvre par des systèmes matériels spéciaux qui exécutent les fonctions ou actes spécifiés ou effectuer des combinaisons de matériel spécial et d'instructions informatiques.

### [Description de l'invention]

Dans la suite de la description, la « mobilité » ou la « démarche » au sens de l'invention correspond à la posture, les mouvements, la locomotion, et l'équilibre de l'utilisateur. L'équilibre correspond notamment à l'équilibre postural lié à la stabilité du corps et plus particulièrement à la stabilité du centre de gravité d'un utilisateur. Néanmoins il peut intégrer aussi bien l'équilibre statique que l'équilibre dynamique.

L'expression « paramètres de posture ou de mobilité » correspond à des paramètres biomécaniques identifiés en position statique ou dynamique.

L'expression « analyse d'un mouvement », « analyse de la mobilité » ou « analyse de la démarche » correspond, au sens de l'invention, à l'attribution d'une ou de plusieurs valeurs par exemple un score, un classement ou une note à une trajectoire ou au déplacement d'un pied d'un utilisateur. Cette caractérisation de la démarche permet d'obtenir une ou plusieurs valeurs numériques ou alphanumériques de paramètres bio-mécaniques représentatifs de la démarche.

L'expression « données brutes » correspond à des données générées par des capteurs et n'ayant pas encore fait l'objet d'une transformation. Cela peut par exemple correspondre aux données générées par une plateforme inertielle. Le traitement des données brutes peut permettre d'obtenir des valeurs de paramètre biomécanique.

Par « paramètre biomécanique », on entend au sens de l'invention une caractéristique de la posture ou de la mobilité de l'utilisateur. Un paramètre biomécanique peut être déterminé par diverses opérations de calcul à partir de valeurs de paramètres de démarche générés par des capteurs d'une semelle connectée. Par « paramètre biomécanique avancé », on entend au sens de l'invention une caractéristique de la posture ou de la mobilité de l'utilisateur déterminé à un moment clé d'un cycle de marche et/ou de course et donc plus complexe à déterminer.

Un cycle pouvant par exemple être un cycle de marche. Il existe différents types d'activités telles que le pas, la montée d'une marche, la descente d'une marche, une foulée, un saut, un plat, un statisme, un piétinement, un agenouillement... De ce fait, un cycle peut également correspondre à une pluralité d'activités de types différents en fonction de la complexité du mouvement réalisé par l'utilisateur

On entend par « semelle » un objet permettant de séparer le pied de l'utilisateur du sol. Une chaussure peut comporter une couche de semelle supérieure en contact direct avec le pied de l'utilisateur et une couche de semelle inférieure en contact direct avec le sol ou plus généralement l'environnement extérieur. Une chaussure peut aussi comporter une semelle interne amovible.

On entend par « sensiblement identique » au sens de l'invention une valeur variant de moins de 30 % par rapport à la valeur comparée, de préférence de moins de 20 %, de façon encore plus préférée de moins de 10 %.

On entend par « amovible » la capacité à être détachée, enlevée ou démontée aisément sans avoir à détruire des moyens de fixation soit parce qu'il n'y a pas de moyen de fixation soit parce que les moyens de fixation sont aisément et rapidement démontables (e.g. encoche, vis, languette, ergot, clips). Par exemple, par amovible, il faut comprendre que l'objet n'est pas fixé par soudure ou par un autre moyen non prévu pour permettre de détacher l'objet.

On entend par « traiter », « calculer », « déterminer », « afficher », « transformer », « extraire », « comparer » ou plus largement « opération exécutable », au sens de l'invention, une action effectuée par un dispositif ou un processeur sauf si le contexte indique autrement. À cet égard, les opérations se rapportent à des actions et/ou des processus d'un système de traitement de données, par exemple un système informatique ou un dispositif informatique électronique, qui manipule et transforme les données représentées en tant que quantités physiques (électroniques) dans les mémoires du système informatique ou d'autres dispositifs de stockage, de transmission ou d'affichage de l'information. Ces opérations peuvent se baser sur des applications ou des logiciels.

Les termes ou expressions « application », « logiciel », « code de programme », et « code exécutable » signifient toute expression, code ou notation, d'un ensemble d'instructions destinées à provoquer un traitement de données pour effectuer une fonction particulière directement ou indirectement (e.g. après une opération de conversion vers un autre code). Les exemples de code de programme peuvent inclure, sans s'y limiter, un sous-programme, une fonction, une application exécutable, un code source, un code objet, une bibliothèque et/ou tout autre séquence d'instructions conçues pour l'exécution sur un système informatique.

Au sens de l'invention le terme « processeur » désigne au moins un circuit matériel configuré pour exécuter des instructions contenues dans le code de programme. Le circuit matériel peut être un circuit intégré. Des exemples d'un processeur comprennent, sans s'y limiter, une unité de traitement central (CPU), un processeur de réseau, un processeur de vecteur, un processeur de signal numérique (DSP), un réseau de grille programmable sur le terrain (FPGA), un ensemble logique programmable (PLA), un circuit intégré spécifique à l'application (ASIC), un circuit logique programmable et un contrôleur.

On entend par « couplé », au sens de l'invention, connecté, directement ou indirectement avec un ou plusieurs éléments intermédiaires. Deux éléments peuvent être couplés mécaniquement, électriquement ou liés par un canal de communication.

Dans la suite de la description, les mêmes références sont utilisées pour désigner les mêmes éléments. En outre, les différentes caractéristiques présentées et/ou revendiquées peuvent être avantageusement combinées. Leur présence dans la description ou dans des revendications dépendantes différentes, n'excluent pas cette possibilité.

Comme cela a déjà été évoqué précédemment, l'invention permet de personnaliser les semelles sur la base de la biomécanique de marche d'un utilisateur, qui pourrait également être combinée à la composition, la matière formant des semelles utilisées par l'utilisateur, afin de proposer des semelles à la forme parfaitement adaptée à l'utilisateur mais également avec des combinaisons de matières de densités et autres rebonds adaptés aux mouvements de l'utilisateur.

Bien que de nombreuses solutions aient été développées pour permettre une personnalisation de semelles à chaque utilisateur, notamment en vue de proposer des produits et/ou services au plus proche de leur besoin, il en résulte que, bien souvent, l'article chaussant n'est pas forcément idéalement agencé pour recevoir la semelle personnalisée. En effet, les paramètres liés à ou aux articles chaussants destinés à recevoir une semelle personnalisée ne sont généralement pas pris en compte pour la conception de la semelle personnalisée. Cependant, de façon générale, et plus particulièrement dans le domaine des sports impliquant une marche ou une course à pied, un utilisateur ou plus communément un sportif cherche généralement à s'équiper avec des articles chaussants les plus adaptés à la façon dont il pratique une discipline sportive. Cela est d'autant plus vrai pour les articles chaussants pour lesquels un choix inadapté peut avoir des conséquences sur le bien-être de l'utilisateur et plus particulièrement sur les performances délivrées par le sportif qui en est équipé. Cela peut même occasionner de lourdes dégradations des pieds dudit sportif. En effet, chaque personne possède une façon bien particulière de se déplacer et il est admis que l'on distingue principalement trois grandes familles de foulées différentes : La foulée universelle, La foulée pronatrice ou sur-pronatrice, La foulée supinatrice ou sous-pronatrice.

Bien souvent, pour pouvoir identifier une foulée supinatrice ou sur-pronatrice, des tests et analysent de la foulée par un spécialiste sont nécessaires. Ainsi, de très nombreux sportifs ignorent quel type de foulée ils pratiquent et si celle-ci est prononcée ou non. S'ajoute à cela, le besoin grandissant de sportifs ou non, d'équiper leur article chaussant avec des semelles orthopédique afin de rectifier ou d'éviter l'apparition d'un défaut dans leur démarche. De ce fait, ils peuvent aisément choisir des articles chaussants peu ou pas adaptés à une semelle personnalisée, pouvant ainsi causer des dégradations de leurs pieds à long terme.

Pour répondre à ce problème, la demanderesse a développé une solution permettant de calculer des valeurs personnalisées de paramètres d'une nouvelle semelle. Ainsi, elle permet la conception de semelles sur mesure automatiquement en particulier en se basant sur des paramètres de posture ou de mobilité du futur utilisateur de la nouvelle semelle et des paramètre de chaussure de l'utilisateur. En outre, la solution peut prendre en compte des paramètres de posture ou de mobilité d'utilisateurs tiers avec leurs paramètres de chaussure de façon à ajuster au mieux les valeurs personnalisées de paramètres de la nouvelle semelle.

**Ainsi, selon un premier aspect**, l'invention porte sur un procédé 500 de calcul de valeurs personnalisées de paramètres d'une nouvelle semelle pour la conception de semelles sur mesure, ledit procédé étant mis en œuvre au sein d'un système informatique configuré de manière idoine.

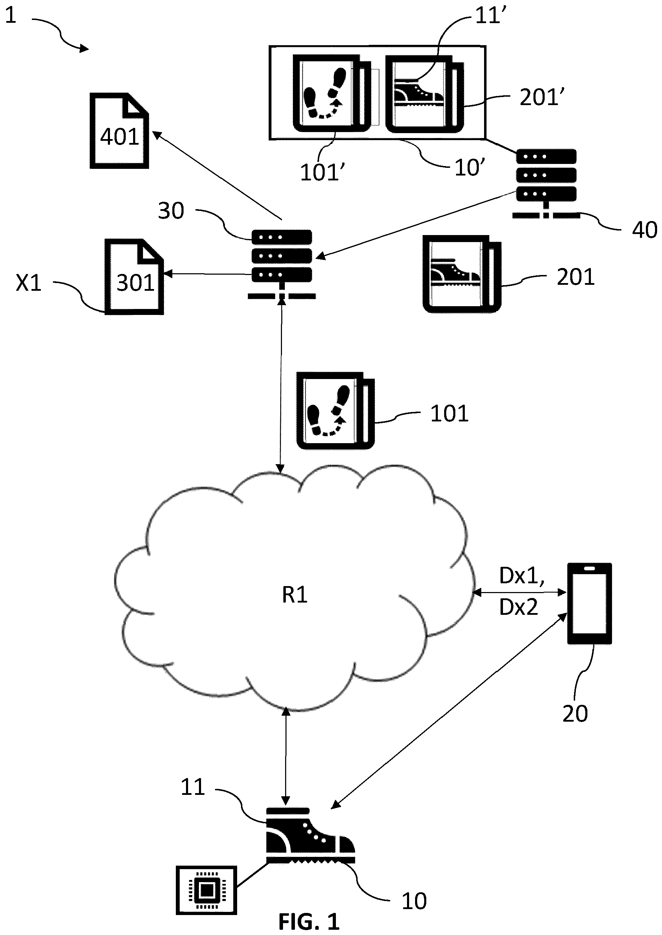

Comme cela a été mentionné, la semelle sur mesure est avantageusement réalisée sur la base de calculs de valeurs personnalisées réalisées à partir de paramètres obtenus au moyen du système illustré par la figure 1. Le système utilise à cette fin une ou deux semelles connectées.

Comme illustré à **la** **figure 1**, un système 1 de calcul de valeurs personnalisées de paramètres d'une nouvelle semelle pour la conception de semelles sur mesure comprend au moins un dispositif informatique de calcul 30 et un article chaussant 11 comprenant une semelle connectée 10.

En outre, un système 1 de calcul de valeurs personnalisées de paramètres d'une nouvelle semelle pour la conception de semelles sur mesure peut également comporter un dispositif informatique de présentation 20 et un dispositif informatique tiers 40.

Ainsi, dans le cadre de l'invention, un système 1 comprend un **dispositif informatique de calcul 30** configuré pour charger des valeurs de paramètres de posture ou de mobilité 101 calculées à partir de données brutes générées par au moins une semelle connectée 10 d'un article chaussant 11 utilisé par un utilisateur, charger des valeurs de paramètres de chaussures 201, calculer une ou plusieurs valeurs personnalisées de paramètres d'une nouvelle semelle 301 à partir des valeurs de paramètres de posture ou de mobilité 101 et des valeurs de paramètres de chaussure 201, recevoir et analyser des informations d'utilisateurs tiers d'articles chaussants tiers équipés de semelles connectées tierces 10', et, identifier des valeurs de paramètres de semelle ajustées.

Un dispositif informatique de calcul 30 comprend avantageusement une unité de traitement ou un processeur, par exemple sous la forme d'un microcontrôleur coopérant avec une mémoire de données, éventuellement une mémoire de programme, lesdites mémoires pouvant être dissociées. Une telle mémoire de données peut être configurée pour stocker un programme d'ordinateur dont les instructions de programme, interprétables et exécutables par l'unité de traitement, permettent d'adapter automatiquement un dispositif informatique conventionnel pour que celui-ci se mue en un dispositif informatique de calcul 30 conforme à l'invention.

La mémoire de données peut être partiellement ou entièrement électriquement effaçable afin d'être mise à jour. Généralement, une section de ladite mémoire de données n'est pas effaçable par construction, ou est protégée contre un tel effacement par un mécanisme de sécurité. Une telle section de mémoire enregistre de manière pérenne, notamment la valeur d'une donnée d'identification unique caractérisant une semelle connectée 10 au regard d'autres semelles connectées. L'unité de traitement coopère avec lesdites mémoires au moyen d'un bus de communication interne. Ainsi, les données issues de capteurs positionnés dans ladite semelle connectée peuvent être stockées dans une telle mémoire de données. Ladite mémoire de données peut en outre stocker des données personnelles associées à l'utilisateur de la semelle connectée. Le dispositif informatique de calcul 30 peut être situé dans un cloud. Ainsi, les données issues du ou des capteurs positionnés dans la semelle connectée 10 peuvent être liées aux données personnelles de l'utilisateur, au travers de la valeur de la donnée d'identification unique de la semelle connectée 10. Les données personnelles associées à l'utilisateur de la semelle connectée 10 peuvent correspondre à des données accessibles sur requête auprès d'un dispositif informatique configuré pour stocker de telle données personnelles. Avantageusement, les données personnelles sont saisies par l'utilisateur de la semelle connectée 10 via une application dédiée installée sur un dispositif informatique de présentation 20. Ainsi, l'utilisateur peut saisir des données personnelles telles que son sexe, son âge, son poids, sa taille, sa pointure ou plus généralement toute donnée morphométrique ou non présentant un intérêt dans le cadre du calcul des valeurs de paramètres de posture ou de mobilité de l'utilisateur. Ainsi, il est prévu que l'utilisateur puisse indiquer, dans le cadre de la saisie de ses informations personnelles, une ou plusieurs pathologies ayant une influence sur sa démarche, ou plus généralement toute défaillance physique impliquant des difficultés à se mouvoir. Une telle pathologie ou une telle défaillance physique peut être sélectionnable via une liste au travers de l'application dédiée ou bien peut être inscrite dans un champ dédié. Une telle pathologie ou une telle défaillance physique peut consister avantageusement mais de manière non limitative à des problèmes articulaires d'un ou plusieurs membres de l'utilisateur, un *hallux valgus,* un *hallux rigidus,* une griffe d'orteil (« orteil en marteau »), une bunionette, le syndrome de Morton, le syndrome algique du 2ème rayon, la bursite inter métatarsienne, les sésamoïdopathies, les tendinopathies ou encore toute blessure physique ayant une incidence sur la démarche de l'utilisateur.

Un tel dispositif informatique de calcul 30 comprend également des moyens de communications configurés pour communiquer au travers d'un réseau de communication R1 de longue portée du type Internet, LoRa ou Sigfox ou de tout autre réseau de communication équivalent. Avantageusement, un dispositif informatique de calcul 30 peut correspondre à un serveur informatique ou encore à un boitier électronique d'une semelle connectée 10. Bien entendu, un dispositif informatique de calcul 30 ne saurait se limiter à un serveur informatique et pourra également correspondre à une machine informatique de type ordinateur comprenant en outre une interface homme-machine, c'est-à-dire tout élément permettant à un être humain de communiquer avec un ordinateur en particulier et sans que cette liste soit exhaustive, un clavier et des moyens permettant en réponse aux ordres entrés au clavier d'effectuer des affichages et éventuellement de sélectionner à l'aide de la souris ou d'un pavé tactile des éléments affichés sur un écran. Un autre exemple de réalisation est un écran tactile permettant de sélectionner directement sur l'écran les éléments touchés par le doigt ou un objet et éventuellement avec la possibilité d'afficher un clavier virtuel.

Un dispositif informatique de calcul 30 peut avantageusement communiquer avec d'autres dispositifs informatiques, tel qu'un dispositif informatique de présentation 20 ou encore avec une semelle connectée 10 équipant un article chaussant 11.

Pour cela, un article chaussant 11 correspond à une chaussure destinée à être portée par un utilisateur. Généralement, l'utilisateur portera deux articles chaussants 11, un à chaque pied. Les articles chaussant 11 seront équipés d'une, de préférence de deux semelles connectées 10 (i.e. une semelle connectée 10 par article chaussant).

En effet, un des avantages de l'invention est de pouvoir se baser sur des données brutes générées par une ou plusieurs semelles connectées 10 afin de pouvoir identifier des valeurs de paramètres de semelle ajustées à l'article chaussant 11.

Une semelle connectée 10 est configurée pour générer des données brutes à partir desquelles il est possible de calculer des valeurs de paramètres de posture ou de mobilité 101. De telles données brutes pourront être envoyées directement au dispositif informatique de calcul 30 qui sera alors configuré pour calculer les valeurs de paramètres de posture ou de mobilité 101 à partir des données brutes reçues et pour les stocker dans sa mémoire de données. Il est également prévu que les données brutes générées par une semelle connectée 10 soient transmises à un dispositif informatique tiers 40 qui sera alors configuré pour calculer les valeurs de paramètres de posture ou de mobilité 101 à partir des données brutes reçues. Les valeurs de paramètres de posture ou de mobilité 101 pourront ainsi être chargées soit directement à partir de la mémoire de données du dispositif informatique de calcul 30 soit à partir du dispositif informatique tiers 40. Dans un mode de réalisation particulier, une semelle connectée 10 pourra comprendre des ressources matérielles et logicielles configurées pour calculer des valeurs de paramètres de posture ou de mobilité 101.

Les valeurs de paramètres de posture ou de mobilité 101 calculées à partir de données brutes issues d'une semelle connectée 10 sont généralement générées en lien avec des paramètres biomécaniques identifiés de l'utilisateur en position dite dynamique, c'est-à-dire que l'utilisateur effectue au moins un mouvement. En complément, les paramètres biomécaniques identifiés de l'utilisateur peuvent être associés à une position dite statique, c'est-à-dire que l'utilisateur n'effectue aucun mouvement. De tels paramètres biomécaniques associés à une position statique peuvent notamment correspondre aux informations personnelles de l'utilisateur saisies en lien avec des données morphométriques du pied (forme, dimensions, pointure etc.) de l'utilisateur. Les valeurs de paramètres de posture ou de mobilité associées à une position statique pourront ainsi être comparées aux valeurs de paramètres de posture ou de mobilité associées à une position dynamique afin de mettre en évidence une concordance ou non entre lesdites valeurs. En effet, en fonction des valeurs de paramètres de posture ou de mobilité associées à une position statique, notamment quand celles-ci révèlent une pathologie, il peut être attendu que les valeurs de paramètres de posture ou de mobilité associées à une position dynamique révèlent un trouble de la démarche associée à ladite pathologie.

En outre, les valeurs de paramètres de posture ou de mobilité peuvent être déterminées à partir d'exercices spécifiques réalisés par l'utilisateur. De tels exercices sont par exemple des étapes de marche ou de montée d'escalier. Ainsi, une valeur de paramètre de posture ou de mobilité de type dynamique peut représenter un mouvement d'un utilisateur tel qu'à titre d'exemple non limitatif, un « pas » et une valeur de paramètre de posture ou de mobilité de type statique peut, de manière avantageuse mais non limitative, représenter une posture de type « à genou » d'un utilisateur. Il existe différents types d'exercices tels que le pas, la montée d'une marche, la descente d'une marche, une foulée, un saut, un plat, un statisme, un piétinement, un agenouillement... De ce fait, il est possible de déterminer une pluralité de valeurs de paramètres de posture ou de mobilité à partir de tels exercices comme notamment le mouvement du pied dans l'article chaussant et ainsi mesurer la rotation de la cheville de l'utilisateur et surtout le niveau de maintien qu'offre ledit article chaussant. On pourrait également demander à la personne de faire d'autres exercices pour identifier plus d'informations sur la souplesse, l'amorti etc. de l'article chaussant.

Les **paramètres de posture ou de mobilité 101** peuvent correspondre à des paramètres biomécaniques. Ainsi, les paramètres de posture ou de mobilité 101 pourront être sélectionnés par exemple parmi : des valeurs de pronation/supination, des valeurs de force d'impact, des valeurs de longueur des pas, des valeurs de temps de contact, des valeurs d'accélération, des valeurs de vitesse angulaire, des valeurs d'orientation de la semelle, une vitesse de propulsion, un taux de fatigue, un angle de Fick, une direction de propulsion et une direction de décélération. De tels paramètres peuvent également correspondre à la longueur des pas, le temps de contact, le temps de vol, la boiterie, la force de propulsion, l'équilibre et plusieurs autres paramètres relatifs à l'utilisateur et décrivant sa démarche, ses postures et ses mouvements.

Avantageusement, les paramètres de posture ou de mobilité 101 les plus pertinents dans le cadre de la présente invention sont : les paramètres liés à la santé de la personne comme le paramètre de force d'impact au contact au sol (en particulier s'il montre une valeur élevée), les paramètres de pronation et/ ou supination (en particulier s'ils montrent une valeur élevée), et/ou une boiterie. En outre le paramètre d'usure de chaussure est également très pertinent, notamment car il peut créer des blessures.

De façon encore plus préférée, les paramètres de posture ou de mobilité 101 comportent au moins : le paramètre de force d'impact le paramètre de pronation et/ou le paramètre de supination.

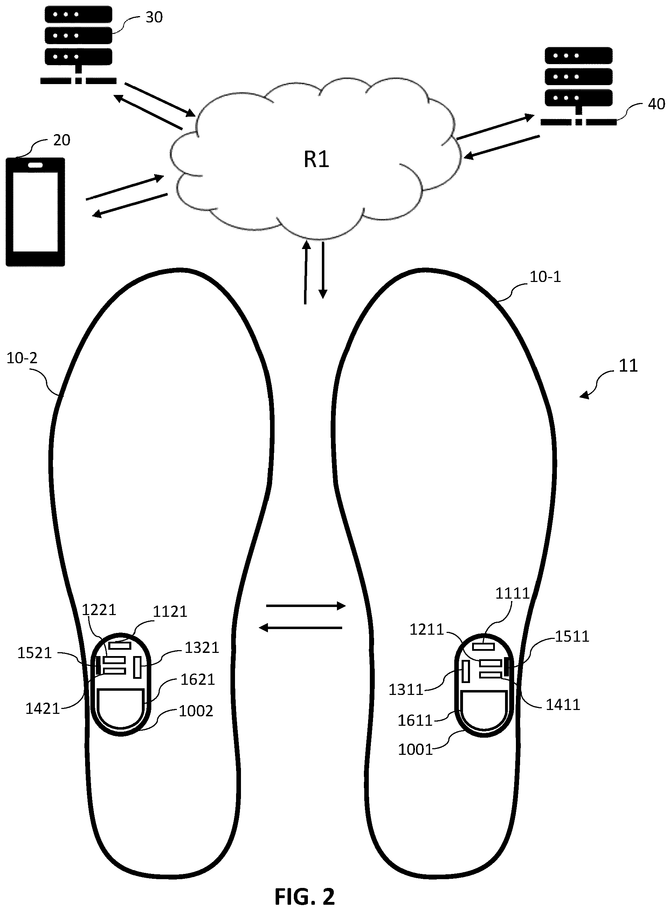

Comme illustré sur la **figure 2**, on partira du principe qu'un article chaussant 11 comprend une paire de chaussures équipée d'une paire de semelles connectées. Dans chacune desdites chaussures, un boitier électronique 1001, 1002 est agencé dans une semelle de chacune desdites chaussures, correspondant ainsi à une semelle connectée référencée 10-1, 10-2 en lien avec la figure 2.

Les semelles connectées peuvent par exemple correspondre à des semelles extérieures ou à des semelles intérieures, d'un article chaussant. Ces semelles peuvent être amovibles ou être intégrées de manière permanente au semelage de l'article chaussant.

Classiquement, les semelles connectées 10-1, 10-2 comportent chacune un boitier électronique 1001,1002. Comme présenté sur la figure 2, le boitier électronique 1001,1002 est de préférence positionné au niveau d'une portion médiane de la semelle.

Un boitier électronique 1001, 1002 ne pèse avantageusement que quelques grammes et présente une dimension réduite adaptée pour une semelle d'une chaussure, ce boitier électronique 1001, 1002 se loge de façon peu encombrante dans n'importe quelle semelle intérieure et/ou extérieure. Ce faible volume limite l'impact sur le confort de l'utilisateur et présente l'avantage d'optimiser les coûts de production en rendant moins onéreux et plus simple l'intégration de cette technologie dans la semelle lors du processus industriel.

Le choix de la matière du boitier électronique 1001, 1002 est réalisé de façon à assurer sa solidité ainsi que la possibilité de l'insérer dans une semelle. En effet, il convient de pouvoir fabriquer un produit qui puisse d'une part, résister au poids d'une personne et, d'autre part, être facilement inséré dans une semelle ou une chaussure. Allier miniaturisation et résistance du boitier est un véritable challenge : il a fallu réaliser de nombreux prototypes avant de déterminer la matière qui permette d'insérer un tel boitier dans une semelle, sans altérer le confort de celle-ci.

Un tel boitier électronique 1001, 1002 comporte une plateforme inertielle 1111,1121 configurée pour générer un ensemble de données (e.g données brutes) sur la démarche d'un utilisateur portant un article chaussant 11 comportant au moins une semelle connectée 10. En particulier, la plateforme inertielle 1111, 1121 est configurée pour générer un ensemble de données sur un mouvement du pied d'un utilisateur équipé de la semelle connectée 10.

Au cours de la marche d'un utilisateur, la plateforme inertielle 1111, 1121 acquière des signaux représentatifs d'un paramètre de mouvement (accélération et/ou vitesse, par exemple vitesse angulaire) du pied selon les axes X, Y, Z. En outre, ces données peuvent ensuite être traitées pour générer au moins un signal d'accélération.

Le boitier électronique 1001, 1002 peut également comporter un ou plusieurs magnétomètres de façon à acquérir trois signaux bruts supplémentaires correspondant aux valeurs de champs magnétiques sur trois dimensions.

Chaque boitier électronique 1001, 1002 peut comporter par ailleurs d'autres capteurs, notamment un inclinomètre, un baromètre, un capteur de température, un capteur d'humidité et un altimètre pour bénéficier d'une précision accrue. En outre, le boitier électronique peut être couplé à d'autres capteurs par exemple répartis dans la semelle tels que des capteurs de pressions ou des capteurs de force. En particulier les capteurs de pressions et/ou de force peuvent comporter des électrodes et être constitué de matériaux piézoélectriques.

La plateforme inertielle est par exemple constituée d'au moins un accéléromètre et un gyroscope. De façon préférée, elle comporte plusieurs accéléromètres et gyroscopes. De façon plus préférée, la plateforme inertielle 1111, 1121 comporte au moins un accéléromètre et au moins un gyroscope, et peut être complétée par d'autres capteurs, notamment un magnétomètre, un baromètre et un altimètre.

En outre, avantageusement, les données générées par les boitiers électroniques 1001, 1002 sont cryptées. Dans ce cas, avantageusement, seul le dispositif informatique destiné à recevoir lesdites données générées est configuré pour les décrypter.

En particulier, les données générées par les boitiers électroniques 1001, 1002 sont cryptées à l'aide de clés publiques chacune associée à un des boitiers électroniques et le dispositif informatique de calcul 30 peut disposer des clés privées nécessaires au décryptage des données générées.

En outre, le boitier électronique 1001, 1002 peut comporter un module de traitement de données 1211,1221 configuré pour transformer l'ensemble des données générées grâce à des algorithmes prédéfinis. Ainsi, les boitiers électroniques 1001, 1002 peuvent être configurés pour réaliser un traitement des signaux générés par la plateforme inertielle de façon à faciliter le traitement ultérieur par un dispositif informatique. Les données reçues via les capteurs situés dans les semelles intérieures et/ou extérieures sont traitées selon un ou plusieurs algorithmes dans chacun des boîtiers électroniques. Le module de traitement est avantageusement configuré pour réaliser un prétraitement des données générées et éventuellement pour réaliser un traitement suffisant pour générer une information sur la posture ou la démarche de l'utilisateur, information que le boîtier électronique transmet à un dispositif informatique, en temps réel ou de manière différée avec les données brutes.

Les boîtiers électroniques 1001, 1002 peuvent également être paramétrés en un boîtier esclave, qui reçoit les données générées par les capteurs situés dans sa semelle/chaussure et les transmet au boîtier maitre, (dénommé aussi boîtier principal), qui reçoit les données du boîtier esclave, les traite en les comparant à ses propres données et génère une information sur la posture de l'utilisateur en général et de ses pieds en particulier, information que le boîtier maitre transmet à un dispositif informatique en temps réel ou de manière différée.

Le module de traitement 1211,1221 permet d'analyser en 3D la posture, les mouvements, la locomotion, l'équilibre et l'environnement de l'utilisateur, et plus généralement tout ce qui sera qualifié comme étant sa marche, à partir des données recueillies par la plateforme inertielle et les éventuels capteurs complémentaires placés dans la semelle.

Le module de traitement 1211, 1221 peut être utilisé pour générer des paramètres biomécaniques de la démarche. Avantageusement, le module de traitement 1211, 1221 est configuré pour transformer l'ensemble de données en au moins un **paramètre de posture ou de mobilité 101** tel que ceux évoqués précédemment.

En outre, la transformation par le module de traitement 1211, 1221 peut avantageusement comprendre la segmentation des données en une pluralité de phases. De préférence, le module de traitement 1211, 1221 de données est apte à segmenter un pas en au moins quatre phases telles que : la phase d'impact (correspond au moment précis du contact du pied avec le sol), la phase d'appui (se déroule depuis la phase d'impact jusqu'au décollement du talon du sol), la phase de propulsion (débute quand le talon a quitté le sol et se termine quand le premier orteil a quitté le sol) et la phase de vol (débute quand le premier orteil a quitté le sol et se termine quand le talon touche le sol).
De façon plus particulière, le découpage ou la segmentation du pas peut permettre d'identifier les zones principales d'appui de l'utilisateur. Ainsi, il est possible de mesurer la forme du pas pendant la marche ou toute autre activité de l'utilisateur afin de déterminer les malformations éventuelles des pieds et postures de l'utilisateur.
Les informations générées vont ensuite être transmises au second boitier électronique 1002, ou plus généralement au boîtier maitre (boîtier principal) par une émission de signaux qui peuvent, à titre d'exemple non limitatif, être de type Bluetooth.
Le boitier électronique 1001, 1002 peut avantageusement être formé par l'encapsulation de ses composants. Par exemple, l'encapsulation peut prendre la forme un revêtement enrobant ou d'une résine (e.g. silicone, époxy, polyuréthane). L'encapsulation de l'intégralité des composants (e.g. plateforme inertielle, module de traitement...) offre une bonne isolation et combine ainsi de bonnes propriétés électriques à une excellente protection mécanique.

Lorsqu'un boitier électronique n'est pas en mesure de communiquer en temps réel avec l'autre boitier et/ou avec le dispositif informatique, il stocke les informations récoltées et les transmettra en différé lorsque l'échange sera de nouveau possible. Cette transmission en différé des données recueillies est rendue possible grâce à la capacité de stockage dont est doté chacun des boitiers électroniques.
Ainsi, le boitier électronique comporte un module de stockage 1311,1321 de données, configuré pour mémoriser au moins une partie des données transformées et/ou des données générées par le module de traitement. Un tel module de stockage 1311,1312 de données peut avantageusement présenter une capacité de mémoire inférieure à 512 ko, de préférence inférieure à 128 ko, de façon plus préférée inférieure à 32 ko et de façon encore plus préférée inférieure à 16 ko. En particulier, le module de stockage peut correspondre à de la mémoire disponible sur un CPU.
En outre, le boitier électronique 1001, 1002 comporte des premiers moyens de communication. Ainsi, en particulier, chacun des boîtiers électroniques est conçu de manière à pouvoir communiquer indépendamment avec l'autre et/ou directement avec un dispositif informatique ou encore avec un dispositif électronique communicant afin de pouvoir échanger ses propres informations sur la posture/le mouvement/l'activité de son pied, dont il a reçu les données via les différents capteurs de la semelle intérieure et/ou extérieure de la chaussure ainsi équipée.
De préférence, le boitier électronique 1001, 1002 comporte des premiers moyens de communication 1411, 1421 configurés de façon à ce que le boitier électronique 1001, 1002 d'au moins une des semelles soit apte à transmettre au moins une partie des données brutes, en temps réel ou en différé, à un dispositif informatique de calcul 30 ou à un dispositif informatique tiers 40 ou encore à un dispositif informatique de présentation 20. Comme présenté, ces données sont préférentiellement des données dites brutes, c'est-à-dire des données telles que générées par la plateforme inertielle (de préférence 9 axes et au moins à 200 Hz), mais peuvent aussi être des données prétraitées ou des valeurs de de paramètres de posture ou de mobilité 101.
Avantageusement, chaque boitier électronique 1001, 1002 comporte des seconds moyens de communication configurés de façon à ce que le boitier électronique 1001 d'une semelle connectée soit apte à communiquer avec le boitier électronique 1002 d'une seconde semelle connectée. Ainsi les boitiers électroniques 1001, 1002 seront capables d'échanger des informations en temps réel. En effet, la génération des données par les plateformes inertielles doit de préférence être synchronisée et cela passe avantageusement par une communication entre les deux boitiers électroniques 1001, 1002. De façon plus préférée, les boitiers électroniques 1001, 1002 sont configurés de façon à pouvoir vérifier ponctuellement leur synchronisation.

En particulier, les deux boitiers électroniques 1001, 1002 sont configurés pour communiquer entre eux et pour initier la génération de données sur le mouvement du pied d'un utilisateur seulement après réception d'un message de l'autre boitier électronique.
Les premiers et seconds moyens de communication sont aptes à recevoir et à transmettre les données sur au moins un réseau de communication R1. De préférence la communication est opérée par l'intermédiaire d'un protocole sans fils tel que wifi, 3G, 4G, 5G et/ou Bluetooth. De préférence le protocole de communication est un protocole BLE ou ANT+. Ces protocoles de communications permettent une consommation faible en énergie.
Avantageusement, chacun des boitiers 1001, 1002 est conçu de manière à pouvoir communiquer avec le second boitier, par exemple par des signaux d'ondes courtes ou hautes fréquences de type Bluetooth Low Energy ou ANT+.
Ainsi, en particulier, chacun des boîtiers, qu'il soit esclave ou Maitre, est conçu de manière à pouvoir communiquer indépendamment avec l'autre et/ou directement avec un dispositif informatique afin de pouvoir échanger ses propres informations sur la posture/le mouvement/l'activité de son pied, dont il a reçu les données via les différents capteurs de sa semelle connectée intérieure et/ou extérieure d'un article chaussant.
Avantageusement, du fait de son confinement à l'intérieur d'un boitier placé sous le corps d'une personne, l'antenne 1511, 1521 doit de préférence être disposée à l'intérieur du boitier sur le côté orienté vers le côté extérieur de la semelle. Ce positionnement de l'antenne est préférable dans la mesure où des tests en laboratoire ont permis d'établir que le signal émis depuis une semelle ou une chaussure est absorbée à 70 % par le corps humain. Cette antenne doit donc être positionnée en périphérie du pied et orientée de telle manière à toujours pouvoir transmettre le signal au boitier de la seconde semelle. De façon préférée, l'antenne peut être une antenne imprimée sur une carte électronique. Alternativement, l'antenne peut être imprimée sur une face intérieure du boitier et connectée à la carte électronique par câblage. L'antenne peut de préférence être positionnée sur une partie basse par rapport à la carte électronique. Ainsi, la carte électronique vient faire contact avec l'antenne.
En outre, le boitier électronique 1001, 1002 comporte une source d'énergie 1611,1621. La source d'énergie est de préférence de type batterie, rechargeable ou non. De préférence la source d'énergie est une batterie rechargeable. En outre, elle peut être associée à un système de recharge par le mouvement ou par une énergie extérieure. Le système de recharge par une énergie extérieure peut notamment être un système de recharge par connexion filaire, un système de recharge par induction ou encore photovoltaïque.

Le boitier électronique 1001, 1002 peut comporter une source d'énergie 1611,1621 de type batterie rechargeable, dont la recharge peut être réalisée selon différentes technologies :
- par chargeur, avec un connecteur affleurant au niveau de la semelle ;
- avec un dispositif de recharge mécanique intégré à la semelle, comme par exemple un dispositif piézoélectrique apte à fournir une énergie électrique à partir de la marche ;
- avec un dispositif sans contact, par exemple par induction ; ou
- avec un dispositif photovoltaïque.

En outre, le boitier électronique selon l'invention peut comporter un moyen de connexion filaire, de préférence protégé par une languette amovible. Une telle languette peut de préférence être réalisée en polymère de type élastomère ou polyuréthane. Ce moyen de connexion filaire peut être par exemple un port USB ou firewire. Avantageusement, le port USB est également résistant à l'eau ou l'humidité. Ce moyen de connexion filaire peut être utilisé comme évoqué ci-dessus pour recharger la batterie mais également pour échanger des données et par exemple mettre à jour le micrologiciel de la carte électronique portant les différents composants du boitier électronique.

Ces différents composants du boitier électronique sont de préférence agencés sur une carte électronique (ou circuit imprimé). En outre, les différents moyens et modules du boitier électronique 1001, 1002 sont représentés de façon distincte sur la figure 2 mais l'invention peut prévoir divers types d'agencement comme par exemple un seul module cumulant l'ensemble des fonctions décrites ici. De même, ces moyens peuvent être divisés en plusieurs cartes électroniques ou bien rassemblés sur une seule carte électronique.

En outre, un système 1 utilisé dans le cadre de l'invention comporte un dispositif informatique de présentation 20 pouvant être configuré pour recevoir des données brutes ou prétraitées, générées par une semelle connectée10 ou plus particulièrement par un boitier électronique 1001, 1002. Le dispositif informatique de présentation 20 est généralement une tablette, un téléphone intelligent mobile (« smartphone » en terminologie anglosaxonne). Il peut être configuré pour transférer ces données à un dispositif informatique de calcul distant. Il est alors par exemple possible d'accéder à ce dispositif informatique distant via une interface web.

Avantageusement, une application dédiée est installée sur le dispositif informatique de présentation 20 afin de traiter les informations transmises par les boitiers et permettre à l'utilisateur d'interagir avec le dispositif informatique en charge du traitement des données brutes générées par la semelle connectée 10. En particulier, l'utilisateur pourra consulter l'ensemble des valeurs de paramètre générées par la semelle connectée ou bien par le dispositif informatique de calcul 30. Ainsi, une semelle connectée 10 peut être associée, de préférence couplée directement ou indirectement à un dispositif informatique de présentation 20.

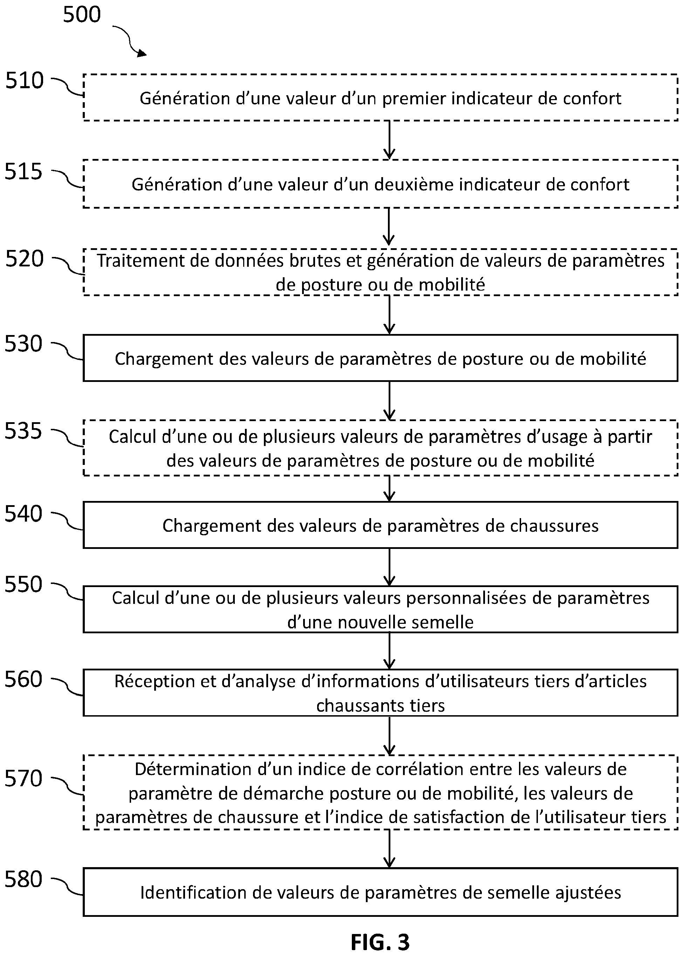

Ainsi, un procédé selon l'invention comporte l'exécution, par un ou plusieurs dispositifs informatiques 20, 30, 40, d'une pluralité d'étapes telles qu'une ou plusieurs des étapes décrites ci-après.
Dans la suite de la description, les étapes d'un procédé conforme à l'invention, notamment décrites en lien avec **la** **figure 3**, sont préférentiellement mises en œuvre par un dispositif informatique de calcul 30.
Plus particulièrement un procédé conforme à l'invention comprend une étape de chargement 530 de valeurs de paramètres de posture ou de mobilité 101 d'un utilisateur, lesdites valeurs de paramètres de posture ou de mobilité ayant été calculées à partir de données brutes générées par au moins une semelle connectée 10 associée à un article chaussant 11 utilisé par ledit utilisateur.
Avantageusement, de telles valeurs de paramètres de posture ou de mobilité sont conservées en mémoire dans la mémoire de données du dispositif informatique de calcul 30. Dans un mode de réalisation particulier il est cependant prévu que les valeurs de paramètres de posture ou de mobilité 101 soient chargées directement auprès d'un dispositif informatique tiers 40 dont la fonction principale serait de générer les valeurs de paramètres de posture ou de mobilité 101 à partir des données brutes générées et envoyées par la semelle connectée 10.
Dans certains cas, il peut être souhaitable d'utiliser certains paramètres de posture ou de mobilité et pas d'autres, par exemple dans le cadre d'un utilisateur présentant une pathologie affectant sa démarche, ou bien dans le cadre d'un sportif pratiquant une activité particulière. Un procédé conforme à l'invention peut comporter une étape de traitement 520, préalable à l'étape de chargement 530, de données brutes générées par l'au moins une semelle connectée 10 utilisée par l'utilisateur. Cette étape de traitement 520 permet avantageusement la génération de valeurs de paramètres de posture ou de mobilité 101. Que les données brutes proviennent d'un dispositif informatique tiers 40 ou qu'elles se trouvent dans la mémoire de données du dispositif informatique de calcul 30, le dispositif informatique de calcul 30 peut être configuré pour, à partir des données brutes, générer les valeurs de paramètres de posture ou de mobilité 101 souhaitées et les plus adaptées à l'utilisateur.

Afin de pouvoir déterminer quels paramètres doivent être pris en compte pour la fabrication d'une nouvelle semelle personnalisée, il peut être avantageux de pouvoir déterminer directement à partir des paramètres de posture ou de mobilité 101, à quel usage ladite nouvelle semelle est destinée. Pour cela, un procédé conforme à l'invention peut comporter une étape de calcul 535 d'au moins une valeur de paramètre d'usage 401 à partir des valeurs de paramètres de posture ou de mobilité 101 générées.
Ainsi, le dispositif informatique de calcul 30 peut comprendre dans une mémoire de données dédiée, un référentiel d'usage. Le référentiel d'usage comporte des valeurs de posture ou de mobilité de référence auxquelles est associé un type d'usage prédéterminé. A titre d'exemples non limitatifs, la détermination d'un usage, par l'étape de calcul 535 d'au moins une valeur de paramètre d'usage 401, peut être associée à un motif de valeurs de posture ou de mobilité 101 de référence spécifique. Une telle détermination d'un usage peut également être assujettie au dépassement ou non d'un seuil prédéterminé. L'homme du métier appréciera que les possibilités de détermination d'un usage en fonction des paramètres de posture ou de mobilité 101 sont très nombreux et qu'il sera possible de configurer le dispositif informatique 30 de calcul pour que lors de la mise en œuvre de l'étape de calcul 535, une ou plusieurs valeurs de paramètre d'usage 401 soit déterminées, en fonction des paramètres de posture ou de mobilité 101 jugés pertinents pour un usage donné. A titre d'exemple non limitatif, une valeur de paramètre d'usage pourra indiquer une utilisation sportive, urbaine, récréative d'un article chaussant 11. Pour cela, le dispositif informatique de calcul 30 peut être configuré pour identifier dans les valeurs de paramètres de posture ou de mobilité 101 préalablement chargées, des valeurs décrivant une force d'impact, une longueur des pas, une d'accélération, une vitesse de propulsion et un temps de vol. Notamment, le dispositif informatique de calcul 30 peut être configuré pour comparer, lors de la mise en œuvre de l'étape de calcul 535, les valeurs décrivant une force d'impact, une longueur des pas, une accélération, une vitesse de propulsion et un temps de vol à une pluralité de motifs de valeurs de référence pour ces paramètres de posture et de mobilité pris seuls ou en combinaison. L'homme du métier comprendra que dans le cadre d'une pratique sportive un ou plusieurs paramètres pourront être pris en compte pour identifier un usage d'un article chaussant. Notamment, une pratique sportive de type course à pied pourra par exemple être caractérisée par des valeurs décrivant une accélération, un temps de vol particulier ou encore une longueur de pas ou bien une vitesse de course ou encore une combinaison de ces paramètres. Dans le cadre de problèmes plutôt liés au domaine de la santé, affectant par exemple la démarche d'un utilisateur, d'autres paramètres de posture ou de mobilité pourront être pris en compte pour identifier un usage donné. Notamment, les paramètres de démarche ou de mobilité, affectant la démarche de l'utilisateur, qui peuvent être analysés sont la présence d'une boiterie, un taux de fatigue des valeurs d'orientation de la semelle l'équilibre. Ainsi, il sera possible de déterminer un usage particulier d'un article chaussant dans le cadre d'une pratique sportive par exemple.

Afin que la nouvelle semelle soit adaptée à l'article chaussant 11 de l'utilisateur, un procédé 500 conforme à l'invention comprend une étape de chargement 540 de valeurs de paramètres de chaussure 201, lesdites valeurs de paramètres de chaussure 201 comportant des valeurs de paramètres structurels et/ou géométriques de l'article chaussant 11 utilisé par l'utilisateur associé à la semelle connectée 10. Les valeurs de paramètres de chaussure 201 peuvent avantageusement être stockées dans une base de données du dispositif informatique de calcul 30 ou bien sur un dispositif informatique tiers 40. En effet, il est prévu que chaque semelle connectée 10 puisse être associée à un article chaussant 11 spécifique. A titre d'exemple non limitatif, le dispositif informatique tiers 40 peut comprendre une base de données indiquant pour chaque semelle connectée 10 à quel modèle d'article chaussant ladite semelle connectée est associée. Il est ainsi possible d'associer des valeurs de paramètres de chaussures à une semelle connectée 10 et par extension d'associer les données générées par ladite semelle connectée à un utilisateur équipé d'un modèle d'article chaussant en particulier. Comme vu précédemment, les valeurs de paramètres de chaussures 201 peuvent comporter des valeurs de paramètres structurels indiquant les différents éléments constituant un modèle d'article chaussant 11 prédéterminé. Les valeurs de paramètres de chaussures 201 peuvent ainsi indiquer la présence d'un contrefort, d'un bout dur, d'une semelle, d'un cambrion, d'une première de montage, d'une première de propreté, d'une tige. Plus particulièrement, les valeurs de paramètres de chaussures 201 peuvent décrire la forme de chacun des éléments constituant l'article chaussant 11, leur agencement mais également elles peuvent décrire des caractéristiques mécaniques pour chaque élément composant l'article chaussant, tel qu'à titre d'exemples non limitatifs une épaisseur de la semelle intercalaire, une résistance à l'abrasion de la semelle extérieure, une rigidité, une isolation, ou encore des propriétés d'amortissement de la semelle intercalaire. Les valeurs de paramètres de chaussures 201 peuvent en outre comporter des valeurs de paramètres géométriques. Lesdites valeurs de paramètres géométriques peuvent indiquer les dimensions relatives à chacun des éléments structurels de l'article chaussant 11. Enfin, les valeurs de paramètres de chaussures 201 peuvent également comporter des valeurs de paramètres esthétiques indiquant par exemple, pour chaque élément structurel une couleur, un type de matériau, un motif esthétique particulier.

Afin de fournir une nouvelle semelle adaptée à l'utilisateur et à l'article chaussant 11 auquel ladite nouvelle semelle est destinée, un procédé 500 conforme à l'invention comprend en outre une étape de calcul 550 d'une ou de plusieurs valeurs personnalisées de paramètres d'une nouvelle semelle 301, la ou lesdites valeurs personnalisées de paramètres de la nouvelle semelle étant calculées à partir des valeurs de paramètres de posture ou de mobilité 101 et des valeurs de paramètres de chaussure 201. En effet, un des avantages de l'invention est de permettre de personnaliser la nouvelle semelle en fonction des valeurs des paramètres de posture ou de mobilité 101 tout en s'assurant que ladite nouvelle semelle puisse s'adapter à l'article chaussant 11 de l'utilisateur. A titre d'exemples non limitatifs, les valeurs de paramètres de posture ou de mobilité 101 peuvent décrire une force d'impact au contact au sol, et une supination. L'étape de calcul 550 peut avantageusement comprendre une comparaison de la valeur associée à la force d'impact, à la valeur de paramètre de chaussure décrivant un élément de l'article chaussant ayant une fonction d'amorti. Ainsi, comme décrit précédemment les valeurs de paramètre de chaussure 201 peuvent comprendre une épaisseur de la semelle intercalaire ou plus généralement des valeurs décrivant des propriétés d'amortissement adaptées à une force d'impact maximale. Dans ce cas, l'étape de calcul 550 permet alors de déterminer que la valeur de paramètre de posture ou de mobilité 101 décrivant la force d'impact de l'utilisateur, est supérieure à la valeur de paramètre de chaussure 201 de l'article chaussant 11 décrivant une force d'impact maximal associée, par exemple à la semelle intercalaire de l'article chaussant 11. L'étape de calcul 550 permet alors de calculer une valeur personnalisée de paramètres d'une nouvelle semelle 301 en lien avec la semelle intercalaire. Cette valeur personnalisée peut correspondre à une augmentation de l'épaisseur de la semelle intercalaire présente dans l'article chaussant 11 par exemple. Pour un utilisateur supinateur de telles valeurs personnalisées, liées à la force d'impact, peuvent ne s'appliquer que sur la partie de la semelle intercalaire pour laquelle un contact du pied avec le sol est détecté, notamment sur la partie extérieure du pied, c'est-à-dire dans la zone voisine au petit orteil, à savoir le *quintus.*

Afin de fournir à l'utilisateur une nouvelle semelle personnalisée, un procédé 500 conforme à l'invention peut comporter une étape de génération 510 d'une valeur d'un premier indicateur de confort Dx1 d'une première semelle, ledit premier indicateur de confort étant généré suite à une saisie de données par l'utilisateur, par l'intermédiaire d'une interface homme-machine d'un dispositif informatique de présentation 20. En effet, il peut être intéressant de disposer de données relatives à l'avis de l'utilisateur concernant l'article chaussant 11 équipé d'une semelle connectée 10 et porté par l'utilisateur. Notamment, il est prévu que l'utilisateur puisse, au travers d'une application dédiée, sélectionner, sur un dispositif informatique de présentation 20, le modèle d'article chaussant correspondant à celui équipé de la semelle connectée. A titre d'exemple non limitatif, l'utilisateur peut avoir accès à la base de données du dispositif informatique tiers 40 précédemment évoquée. Une fois l'article chaussant 11 sélectionné, l'utilisateur peut attribuer, pour chaque valeur de paramètre de chaussure 201 de l'article chaussant 11, une note, c'est-à-dire une valeur numérique, par exemple comprise entre 0 et 5 ou encore à une valeur décimale sous forme de fraction. A partir des valeurs saisies par l'utilisateur, une valeur d'un premier indicateur de confort est générée 510, un tel indicateur peut notamment correspondre à une moyenne, pondérée pour chaque valeur de paramètres de chaussure 201 ou non, calculée à partir des valeurs saisies par ledit utilisateur.

Lors de l'étape de calcul 550 d'une ou de plusieurs valeurs personnalisées de paramètres de la nouvelle semelle 301, ladite étape de calcul peut alors permettre de prendre en compte la valeur générée du premier indicateur de confort. De manière préférée, l'étape de calcul 550 peut consister à calculer une ou plusieurs valeurs personnalisées de paramètres de la nouvelle semelle 301 pour les valeurs de paramètres de chaussure dont la valeur attribuée par l'utilisateur est inférieure à un seuil prédéterminé et à conserver les valeurs de paramètres de chaussure dont la valeur attribuée par l'utilisateur est supérieure à un seuil prédéterminé. Ainsi, seules les valeurs de paramètres de chaussure 201 considérées comme inadaptées pour l'utilisateur peuvent être modifiées afin de fournir à ce dernier une nouvelle semelle hautement personnalisée.
Une fois la valeur du premier indicateur de confort Dx1 d'une première semelle générée 510, un procédé 500 conforme à l'invention peut comporter une étape de génération 515 d'une valeur d'un deuxième indicateur de confort Dx2 d'une deuxième semelle, ledit deuxième indicateur de confort étant généré suite à une saisie de données par l'utilisateur de ladite deuxième semelle, par l'intermédiaire de l'interface homme-machine du dispositif informatique de présentation 20. En effet, il peut être intéressant de disposer de données relatives à l'avis de l'utilisateur concernant un deuxième article chaussant, préférentiellement porté par l'utilisateur. Notamment, il est prévu que l'utilisateur puisse, au travers d'une application dédiée, sélectionner, sur un dispositif informatique de présentation 20, un autre modèle d'article chaussant. Tel qu'évoqué précédemment en lien avec le premier indicateur de confort Dx1, l'utilisateur peut avoir accès à la base de données du dispositif informatique tiers 40. Une fois le deuxième article chaussant sélectionné, l'utilisateur peut attribuer, pour chaque valeur de paramètre de chaussure du deuxième article chaussant, une note, c'est-à-dire une valeur numérique, par exemple comprise entre 0 et 5 ou encore à une valeur décimale sous forme de fraction. A partir des valeurs saisies par l'utilisateur, une valeur d'un deuxième indicateur de confort Dx2 est générée 515, un tel indicateur peut notamment correspondre à une moyenne, pondérée pour chaque valeur de paramètres de chaussure ou non, calculée à partir des valeurs saisies par ledit utilisateur.
Lors de l'étape de calcul 550 d'une ou de plusieurs valeurs personnalisées de paramètres de la nouvelle semelle 301, ladite étape de calcul peut alors permettre de prendre en compte la valeur générée du deuxième indicateur de confort. De manière préférée, l'étape de calcul 550 peut consister à calculer une ou plusieurs valeurs personnalisées de paramètres de la nouvelle semelle 301 pour les valeurs de paramètres de chaussure 201 dont la valeur attribuée par l'utilisateur en lien avec le premier indicateur de confort Dx1 est inférieure à un seuil prédéterminé. L'étape de calcul 550 peut alors permettre d'utiliser les valeurs de paramètres de chaussure du deuxième article chaussant dont la valeur attribuée par l'utilisateur est supérieure à un seuil prédéterminé pour calculer la ou les plusieurs valeurs personnalisées de paramètres de la nouvelle semelle 301.
Ainsi, les valeurs de paramètres de chaussure d'un deuxième article chaussant considérées comme adaptées pour et par l'utilisateur peuvent être utilisées afin de fournir à ce dernier une nouvelle semelle hautement personnalisée.

Un des objectifs de l'invention est de fournir une semelle personnalisée directement adaptée à l'article chaussant 11 de l'utilisateur. Pour cela, un procédé 500 conforme à l'invention comprend une étape de réception et d'analyse 560 d'informations d'utilisateurs tiers d'articles chaussants tiers 11', lesdites informations d'utilisateurs tiers comportant pour chaque utilisateur tiers d'articles chaussants tiers :
∘ des valeurs de paramètres de posture ou de mobilité 101' tiers calculées à partir de données brutes générées par au moins une semelle connectée tierce 10' associée à un article chaussant tiers 11', et
∘ des valeurs de paramètres de chaussure tiers 201' de l'article chaussant tiers 11' associé à l'au moins une semelle connectée tierce 10'.
A l'instar des valeurs de paramètres de posture ou de mobilité 101, des valeurs de paramètres de chaussure 201 associé à la semelle connectée 10, il est prévu qu'un des dispositifs informatique 20, 30, 40 puisse stocker en mémoire une base de données référençant une pluralité d'informations associées à des utilisateurs tiers. Dans un mode de réalisation décrit en lien avec la figure 1, le dispositif informatique de calcul 30 peut accéder directement à la base de données, au travers du réseau de communication R1, hébergée par le dispositif informatique tiers 40.
Une fois les informations d'utilisateurs tiers récupérées, un procédé 500 selon l'invention comprend une étape d'identification 580 de valeurs de paramètres de semelle ajustées, ladite étape d'identification comportant une comparaison des valeurs de paramètres de posture ou de mobilité 101 générées par la semelle connectée 10 et des paramètres de chaussure 201 de l'article chaussant 11 associé à ladite semelle connectée aux informations d'utilisateurs tiers préalablement analysées. Une telle étape 580 permet avantageusement d'identifier une pluralité d'articles chaussants présentant des valeurs de paramètres de chaussure 201' tiers sensiblement identiques aux valeurs de paramètres de chaussure 201 associées aux valeurs de paramètres de posture ou de mobilité 101' tiers. Il est donc possible d'identifier des valeurs de paramètres de chaussure 201' tiers susceptibles d'être les plus adaptées aux valeurs personnalisées de paramètres de la nouvelle semelle 301. En effet, il peut être avantageux de modifier les valeurs personnalisées de paramètres de la nouvelle semelle 301 afin de les ajuster au mieux à l'article chaussant 11 auquel ladite nouvelle semelle est destinée. Comme vu précédemment, une valeur personnalisée de paramètres d'une nouvelle semelle 301 peut correspondre à une augmentation de l'épaisseur de la semelle intercalaire présente dans l'article chaussant 11 par exemple. Cependant, l'augmentation de l'épaisseur de la semelle intercalaire peut être inadaptée à l'article chaussant 11 ou tout au moins générer un inconfort pour l'utilisateur lors de l'utilisation de l'article chaussant 11 et occasionner des blessures lors de leur utilisation.

Pour inclure des données relatives à l'expérience utilisateur relatives à chaque article chaussant 11' tiers, lesdites informations d'utilisateurs tiers peuvent en outre comporter une valeur d'indice de satisfaction d'un utilisateur tiers, de préférence vis-à-vis de la semelle connectée 10' tierce et/ou de l'article chaussant 11' comportant ladite semelle connectée tierce. Une telle valeur d'indice de satisfaction peut consister en une note attribuée par l'utilisateur tiers, c'est-à-dire une valeur numérique, par exemple comprise entre 0 et 5 ou encore à une valeur décimale sous forme de fraction.
Dans ce cas, un procédé 500 conforme à l'invention peut comprendre une étape de détermination 570 d'un indice de corrélation, pour chaque semelle connectée 10' tierce, entre les valeurs de paramètre de posture ou de mobilité 101' tiers, les valeurs de paramètres de chaussure 201' tiers et l'indice de satisfaction de l'utilisateur tiers. Pour cela, une telle étape de détermination 570 peut consister à comparer les valeurs de paramètre de posture ou de mobilité 101' tiers à des valeurs de paramètre de posture ou de mobilité de référence. Les valeurs de paramètre de posture ou de mobilité de référence correspondent en particulier à des valeurs prédéterminées pour des valeurs de paramètres de chaussure de référence en lien avec article chaussant donné. Ainsi, à partir des valeurs de paramètres de chaussure 201' tiers, il est possible de déterminer des valeurs de paramètre de posture ou de mobilité de référence, puis de les comparer aux valeurs de paramètre de posture ou de mobilité 101' tiers. Si les valeurs de paramètre de posture ou de mobilité de référence sont sensiblement identiques aux valeurs de paramètre de posture ou de mobilité 101' tiers alors une corrélation est établie lors de l'étape de détermination 570 entre les valeurs de paramètre de posture ou de mobilité 101' tiers, les valeurs de paramètres de chaussure 201' tiers et l'indice de satisfaction de l'utilisateur tiers.
Alternativement, l'étape de détermination 570 d'un indice de corrélation peut consister à comparer la valeur de paramètre d'usage 401 calculée lors de l'étape de calcul 535, telle que décrit précédemment, à une valeur de paramètre d'usage de référence associée à l'article chaussant 11. Si la valeur de paramètre d'usage 401 est sensiblement identique à la valeur de paramètre d'usage de référence alors une corrélation est établie.
L'établissement d'une corrélation peut ainsi permettre de ne prendre en compte que les indices de satisfaction d'utilisateurs tiers pertinents, c'est à dire pour lesquels l'usage de l'article chaussant associé est en adéquation avec l'utilisation préconisée par le fabricant.

Afin de fournir une nouvelle semelle 301 la plus adaptée à l'article chaussant 11 de l'utilisateur, l'étape 580 peut avantageusement conduire à l'identification d'un changement de matériau présentant des propriétés d'amortissement adaptées à la force d'impact de l'utilisateur en lieu et place de l'augmentation de l'épaisseur évoquée précédemment.
Dans le cas où une telle identification a lieu, un procédé 500 conforme à l'invention est avantageusement configuré pour mettre en œuvre l'étape de calcul 550 d'une ou de plusieurs valeurs personnalisées de paramètres d'une nouvelle semelle 301, ladite étape de calcul 550 comprend en outre une comparaison des valeurs de paramètres de posture ou de mobilité 101 générées et des valeurs de paramètres de chaussure 201 préalablement chargées aux informations utilisateurs préalablement analysées. Les informations utilisateurs peuvent par exemple correspondre à des données physionomiques, physiologiques, ou encore à des préférence ou des habitudes (e.g. sports pratiqué, niveau d'activité, préférence d'amorti...).

Afin de n'utiliser que les informations d'utilisateurs tiers les plus pertinentes, c'est-à-dire celles des utilisateurs tiers présentant une utilisation sensiblement identique à celle de l'utilisateur de l'article chaussant 11 équipé de la semelle connectée 10, l'étape d'identification 580 de valeurs de paramètres de semelle ajustées d'un procédé 500 conforme à l'invention peut prendre en compte l'au moins une valeur de paramètres d'usage calculée lors de l'étape 535. Dans ce cas-là, lors de la réception et de l'analyse 560 d'informations d'utilisateurs tiers d'articles chaussants tiers 11', l'étape de calcul 535 d'au moins une valeur de paramètre d'usage d'un procédé 500 conforme à l'invention peut avantageusement être mise en œuvre à partir des valeurs de paramètres de posture ou de mobilité tiers 101' calculées à partir de données brutes générées par au moins une semelle connectée tierce 10' associée à un article chaussant tiers 11'. A l'instar de la valeur de paramètre d'usage, une valeur de paramètre d'usage tierce est déterminée en lien avec chaque information d'utilisateur.

Pour rester au plus proche de l'utilisation faite pour un article chaussant par l'utilisateur, l'étape d'identification 580 de valeurs de paramètres de semelle ajustées peut comporter l'utilisation d'un référentiel précisant des valeurs cibles ou des plages cibles pour les valeurs personnalisées de paramètres de la nouvelle semelle 301 en fonction des valeurs de paramètres de posture ou de mobilité 101 générées et des valeurs de paramètres de chaussure 201 acquis. Cela permet avantageusement de borner les valeurs personnalisées de paramètres de la nouvelle semelle 301 et d'éviter, par l'utilisation dudit référentiel, de sélectionner des valeurs personnalisées aberrantes ou tout au moins inadaptées. Pour cela un tel référentiel peut comprendre des valeurs de paramètres de posture ou de mobilité de référence et des valeurs de paramètre de chaussure de référence et des écarts de référence minimal et maximal pour chacune desdites valeurs. En effet, et notamment dans le cadre de la comparaison avec les informations d'utilisateurs tiers, ces dernières peuvent comprendre une ou plusieurs valeurs de paramètres de posture ou de mobilité tiers 101' et une ou plusieurs valeurs de paramètres de chaussure tiers 201' de l'article chaussant tiers qui pourraient être considérées comme utilisables ou non. L'utilisation d'un tel référentiel permet de définir des valeurs ou des plages de valeurs pour lesquelles une valeur personnalisée de paramètre de la nouvelle semelle 301 pourra être utilisée. Ainsi, une étape de calcul 550 d'un procédé 500 conforme à l'invention pourra avantageusement permettre d'optimiser une ou plusieurs valeurs personnalisées de paramètres d'une nouvelle semelle 301, sur la base d'un écart entre les valeurs de paramètres de posture ou de mobilité 101 générées et les valeurs de paramètres de posture ou de mobilité de référence et/ou sur la base d'un écart entre les valeurs personnalisées de paramètres de chaussure 201 acquis et les valeurs personnalisées de paramètres d'une nouvelle semelle 301. Par exemple, si une valeur de paramètre de posture ou de mobilité 101 ou une valeur personnalisée de paramètre de la nouvelle semelle 301, n'est pas sensiblement identique à une valeur de paramètre de posture ou de mobilité de référence ou à une valeur de paramètre de chaussure de référence, mais que ladite valeur est comprise dans un écart de référence minimal ou maximal correspondant, alors la valeur personnalisée de paramètre de la nouvelle semelle 301 est conservée. Dans le cas où ladite valeur n'est pas comprise dans un écart de référence minimal ou maximal correspondant, c'est la valeur de référence correspondante qui est utilisée en lieu et place de la valeur personnalisée de paramètre de la nouvelle semelle 301 calculée.
Afin de ne prendre en considération que les informations d'utilisateurs tiers les plus pertinentes, l'étape d'identification 580 de valeurs de paramètres de semelle ajustées d'un procédé selon l'invention peut n'être mise en œuvre uniquement que pour les informations d'utilisateurs tiers comportant une valeur d'indice de satisfaction et/ou un indice de corrélation respectivement supérieur(s) à un seuil prédéterminé. Pour cela, l'étape d'identification 580 peut avantageusement comprendre une opération de filtrage des informations d'utilisateurs tiers en fonction d'une valeur d'indice de satisfaction seuil prédéterminé. Les informations d'utilisateurs tiers ainsi filtrées peuvent ensuite faire l'objet d'une seconde opération de filtrage, lors de l'étape d'identification 580, en fonction de l'indice de corrélation.

Afin de faciliter la fabrication d'une nouvelle semelle notamment par des techniques d'impression 3D, un procédé selon l'invention comporte une étape de génération, à partir des une ou plusieurs valeurs personnalisées de paramètres de la nouvelle semelle 301, d'une maquette numérique de la nouvelle semelle. La maquette numérique selon la présente invention peut être un modèle tridimensionnel de la nouvelle semelle comprenant une description fonctionnelle. La description fonctionnelle peut inclure des informations telles que les matériaux utilisés, où lesdits matériaux sont placés au niveau de la nouvelle semelle.
La maquette numérique peut être un modèle tridimensionnel d'un article chaussant incluant la nouvelle semelle, comme par exemple une maquette numérique dans un système de Conception Assisté par Ordinateur. La maquette numérique peut comprendre des informations sur les matériaux utilisés, leur forme et leurs dimensions.
La maquette numérique de la nouvelle semelle peut également être personnalisée en fonction de données entrées par l'utilisateur. Par exemple, il peut être demandé à l'utilisateur quel (s) capteur (s) la nouvelle semelle personnalisée doit comprendre.

Comme évoqué précédemment, les données brutes sont généralement générées pour une période de temps donnée et en fonction de la démarche d'un utilisateur. L'analyse de la démarche pourrait s'étendre sur la durée de vie de la chaussure ou de l'utilisateur. Dans ce cas, les tendances à long terme, les écarts et les changements du cycle de marche de l'utilisateur au cours de celui-ci peuvent être déterminés.
Ainsi, la semelle connectée 10 peut également être utilisée dans le contexte d'un suivi de la démarche de l'utilisateur, au quotidien. À cette fin, la semelle connectée 10 peut transmettre les données brutes issues des capteurs à un dispositif informatique tels que l'un de ceux déjà évoqués. Une fois les données brutes traitées, les valeurs de paramètres de posture ou de mobilité 101 peuvent ensuite être présentées à l'utilisateur comme un enregistrement de ses activités quotidiennes sur un dispositif informatique de présentation 20. Par exemple, le nombre de pas effectués au cours d'une journée pourrait être extrait des données du capteur et présenté à la personne. En outre, il est possible de calculer la dépense énergétique à partir des données brutes appropriées et de présenter la dépense énergétique quotidienne à la personne. D'autres informations pouvant être présentées à la personne dans le cadre d'un suivi d'activité quotidien incluent la distance de marche et / ou de course, le temps de marche et / ou de course, la vitesse la plus rapide de la journée, etc.
Dans le cadre d'un service de suivi d'activité quotidien, il est également possible de mettre en œuvre un suivi de poids sur la base des données brutes issues de capteurs idoines de la semelle connectée 10. Par exemple, les données brutes du capteur pourraient inclure des informations de pression provenant d'un capteur de pression qui pourraient être utilisées pour déterminer le poids de l'utilisateur. Il est alors possible de présenter le poids journalier à l'utilisateur.
Sur la base du poids déterminé, une semelle intermédiaire et / ou une semelle extérieure spécifique avec des propriétés ou des matériaux spécifiques pourraient être modifiées ou choisies. Par exemple, l'épaisseur de la semelle intermédiaire pourrait être adaptée au poids de l'utilisateur et plus la personne est lourde, plus la semelle intermédiaire pourrait être épaisse pour assurer un amorti suffisant. La semelle extérieure pourrait être rendue plus résistante à l'abrasion si la personne est plutôt lourde pour contrer l'usure au cours du temps.

**Selon un autre aspect, l'invention porte sur** un procédé 600 de fabrication d'une nouvelle semelle sur mesure par un dispositif d'impression en trois dimensions, ledit procédé comportant une étape de téléchargement 610, par le dispositif d'impression, d'un fichier de configuration X1 comportant une ou plusieurs valeurs personnalisées de paramètres de la nouvelle semelle 301 calculées selon un procédé 500 de calcul de valeurs personnalisées de paramètres d'une nouvelle semelle conforme à l'invention. Un tel fichier de configuration est transmis par le dispositif informatique de calcul 30 et peut prendre la forme d'un fichier de format STL (pour STereo-lithographie). Il est également prévu que le procédé 500 de calcul de valeurs personnalisées de paramètres d'une nouvelle semelle puisse comporter une étape de transmission du fichier de configuration X1 directement au dispositif d'impression en trois dimensions et que, à réception dudit fichier de configuration X1 par le dispositif d'impression en trois dimensions, le procédé 600 de fabrication d'une nouvelle semelle soit mis en œuvre.

Un procédé 600 selon l'invention comprend en outre une étape de génération 620, à partir des valeurs personnalisées de paramètres de la nouvelle semelle 301, d'une maquette numérique de la nouvelle semelle. Ainsi, la nouvelle semelle peut être présentée à l'utilisateur ou au fabricant. Par exemple, une vue 3D du modèle de la nouvelle semelle peut être présentée sur un écran d'affichage, à titre d'exemple non limitatif dans une fenêtre d'un navigateur Web ou un tout autre programme approprié. Il est également possible de présenter une telle maquette numérique via une application dédiée accessible depuis un dispositif informatique de présentation 20.

Une fois la maquette numérique générée, un procédé 600 conforme à l'invention comprend une étape d'impression 630 d'une nouvelle semelle à partir de la maquette numérique générée. L'étape d'impression 630 est avantageusement réalisée par frittage sélectif par laser ou encore par photopolymérisation de résines photosensibles avec des rayons UV, ou plus généralement via toute imprimante en trois dimensions adaptée.

La semelle ainsi fabriquée pourra ou non être une semelle connectée. Un boitier électronique tel que les boitiers 1001 ou 1002 pourra alors être prévu pour équiper la semelle sur mesure.

## Revendications

1. Procédé (500) de calcul de valeurs personnalisées de paramètres d'une nouvelle semelle pour la conception de semelles sur mesure, ledit procédé de calcul comportant l'exécution, par un ou plusieurs dispositifs informatique (20, 30, 40), des étapes suivantes :
- une étape de chargement (530) de valeurs de paramètres de posture ou de mobilité (101) d'un utilisateur, lesdites valeurs de paramètres de posture ou de mobilité ayant été calculées à partir de données brutes générées par au moins une semelle connectée (10) associée à un article chaussant (11) utilisé par ledit utilisateur ;
- une étape de chargement (540) de valeurs de paramètres de chaussure (201), lesdites valeurs de paramètres de chaussure (201) comportant des valeurs de paramètres structurels et/ou géométriques de l'article chaussant (11) utilisé par l'utilisateur, associé à la semelle connectée (10) ;
- une étape de calcul (550) d'une ou de plusieurs valeurs personnalisées de paramètres d'une nouvelle semelle (301), la ou lesdites valeurs personnalisées de paramètres de la nouvelle semelle étant calculées à partir des valeurs de paramètres de posture ou de mobilité (101) et des valeurs de paramètres de chaussure (201) ;
- une étape de réception et d'analyse (560) d'informations d'utilisateurs tiers d'articles chaussants tiers (11'), lesdites informations d'utilisateurs tiers comportant pour chaque utilisateur tiers d'articles chaussants tiers :
∘ des valeurs de paramètres de posture ou de mobilité tiers (101 ') calculées à partir de données brutes générées par au moins une semelle connectée tierce (10') associée à un article chaussant tiers (11'), et
∘ des valeurs de paramètres de chaussure tiers (201') de l'article chaussant tiers (11') associé à l'au moins une semelle connectée (10') tierce ;
- une étape d'identification (580) de valeurs de paramètres de semelle ajustées, ladite étape d'identification comportant une comparaison des valeurs de paramètres de posture ou de mobilité (101) générées par la semelle connectée (10) et des paramètres de chaussure (201) de l'article chaussant (11) associé à ladite semelle connectée, aux informations d'utilisateurs tiers préalablement analysées.

2. Procédé (500) de calcul selon la revendication précédente, **caractérisé en ce que** lesdites informations d'utilisateurs tiers comportent en outre une valeur d'indice de satisfaction d'un utilisateur tiers, de préférence vis-à-vis de la semelle connectée (10') tierce et/ou de l'article chaussant (11') comportant ladite semelle connectée tierce.

3. Procédé (500) de calcul selon la revendication 2, **caractérisé en ce que** ledit procédé comprend une étape de détermination (570) d'un indice de corrélation, pour chaque semelle connectée (10') tierce, entre les valeurs de paramètre de posture ou de mobilité (101') tiers, les valeurs de paramètres de chaussure (201') tiers et l'indice de satisfaction de l'utilisateur tiers.

4. Procédé (500) de calcul selon l'une des revendications 2 ou 3, caractérisé en ce l'étape d'identification (580) de valeurs de paramètres de semelle ajustées est mise en œuvre uniquement pour les informations d'utilisateurs tiers comportant une valeur d'indice de satisfaction et/ou un indice de corrélation respectivement supérieur(s) à un seuil prédéterminé.

5. Procédé (500) de calcul selon l'une quelconque des revendications 1 à 4, **caractérisé en ce qu'**il comporte une étape de traitement (520), préalable à l'étape de chargement (530), de données brutes générées par l'au moins une semelle connectée (10) utilisée par l'utilisateur, ladite étape de traitement permettant la génération de valeurs de paramètres de posture ou de mobilité (101).

6. Procédé (500) de calcul selon l'une quelconque des revendications 1 à 5, **caractérisé en ce que** les paramètres de posture ou de mobilité (101) sont sélectionnés parmi la force d'impact au contact au sol, les paramètres de pronation et/ ou supination et/ou une boiterie.

7. Procédé (500) de calcul selon l'une quelconque des revendications 1 à 6, **caractérisé en ce que** ledit procédé comporte une étape de génération (510) d'une valeur d'un premier indicateur de confort (Dx1) d'une première semelle, ledit premier indicateur de confort étant généré suite à la saisie de données par l'utilisateur, par l'intermédiaire d'une interface homme-machine d'un dispositif informatique (20) de présentation et **en ce que** l'étape de calcul (550) d'une ou de plusieurs valeurs personnalisées de paramètres de la nouvelle semelle (301), prend en compte la valeur générée du premier indicateur de confort.

8. Procédé (500) de calcul selon la revendication 7, **caractérisé en ce qu'**il comporte une étape de génération (515) d'une valeur d'un deuxième indicateur de confort (Dx2) d'une deuxième semelle, ledit deuxième indicateur de confort étant généré suite à la saisie de données par l'utilisateur de ladite deuxième semelle, par l'intermédiaire de l'interface homme-machine du dispositif informatique de présentation (20) et **en ce que** l'étape de calcul (550) d'une ou de plusieurs valeurs personnalisées de paramètres de la nouvelle semelle (301), prend en compte la valeur générée du deuxième indicateur de confort.

9. Procédé (500) de calcul selon l'une quelconque des revendications 1 à 8, **caractérisé en ce que** l'étape de calcul (550) d'une ou de plusieurs valeurs personnalisées de paramètres de la nouvelle semelle (301), comprend en outre une comparaison des valeurs de paramètres de posture ou de mobilité (101) et des valeurs de paramètres de chaussure (201) préalablement chargées aux informations d'utilisateurs tiers.

10. Procédé (500) de calcul selon l'une quelconque des revendications 1 à 9, **caractérisé en ce que** l'étape d'identification (580) de valeurs de paramètres de semelle ajustées comporte l'utilisation d'un référentiel précisant des valeurs cibles ou des plages cibles pour les valeurs personnalisées de paramètres de la nouvelle semelle (301) en fonction des valeurs de paramètres de posture ou de mobilité (101) générées et des valeurs de paramètres de chaussure (201) acquis.

11. Procédé (600) de calcul selon l'une quelconque des revendications 1 à 10, **caractérisé en ce qu'**il comporte une étape de calcul (535) d'au moins une valeur de paramètre d'usage (401) à partir des valeurs de paramètres de posture ou de mobilité (101) et **en ce que** l'étape d'identification (580) de valeurs de paramètres de semelle ajustées prend en compte l'au moins une valeur de paramètre d'usage calculée.

12. Procédé (600) de fabrication d'une nouvelle semelle sur mesure par un dispositif d'impression en trois dimensions, ledit procédé comportant les étapes suivantes :
- Téléchargement (610), par le dispositif d'impression, d'un fichier de configuration (X1) comportant une ou plusieurs valeurs personnalisées de paramètres de la nouvelle semelle (301) calculées selon l'une quelconque des revendications 1 à 11 ;
- Génération (620), à partir des valeurs personnalisées de paramètres de la nouvelle semelle (301), d'une maquette numérique de la nouvelle semelle ;
- Impression (630) d'une nouvelle semelle à partir de la maquette numérique générée.

13. Système de calcul de valeurs personnalisées de paramètres d'une nouvelle semelle (301) pour la conception de semelles sur mesure, ledit système comprenant au moins un dispositif informatique de calcul (30) et un article chaussant (11) comprenant une semelle connectée (10), ledit au moins un dispositif informatique de calcul (30) étant configuré pour charger des valeurs de paramètres de posture ou de mobilité (101) calculées à partir de données brutes générées par au moins une semelle connectée (10) d'un article chaussant (11) utilisé par un utilisateur, charger des valeurs de paramètres de chaussures (201), calculer une ou plusieurs valeurs personnalisées de paramètres d'une nouvelle semelle (301) à partir des valeurs de paramètres de posture ou de mobilité (101) et des valeurs de paramètres de chaussure (201), recevoir et analyser des informations d'utilisateurs tiers d'articles chaussants tiers (11') équipés de semelles connectées tierces (10'), et, identifier des valeurs de paramètres de semelle ajustées.

14. Programme informatique comprenant des instructions de programme qui, lorsqu'elles sont exécutées par une unité de traitement d'un dispositif informatique provoquent la mise en œuvre d'un procédé de calcul de valeurs personnalisées de paramètres d'une nouvelle semelle pour la conception de semelles sur mesure selon l'une des revendications 1 à 11
